# FASCICULE DE BREVET EUROPEEN

(11) **EP 2 961 437 B1**
(45) Date de publication et mention de la délivrance du brevet: **12.10.2016**
(21) Numéro de dépôt: 14706009.9
(22) Date de dépôt: 20.02.2014
(51) Int. Cl.: A61L 2/10, B29C 49/42

(54) **PROCEDE DE DECONTAMINATION PAR IRRADIATION DE L'INTERIEUR D'UNE PREFORME**
VERFAHREN ZUR DEKONTAMINATION DES INNEREN EINES VORFORMLINGS DURCH BESTRAHLUNG
PROCESS FOR DECONTAMINATING BY IRRADIATION THE INTERIOR OF A PREFORM

(30) Priorité: 26.02.2013 FR 1351682
(43) Date de publication de la demande: 06.01.2016
(73) Titulaire: Sidel Participations, 76930 Octeville sur Mer (FR)
(72) Inventeur: BELLEC, Caroline, F-76930 Octeville sur Mer (FR); FEUILLOLEY, Guy, F-76930 Octeville sur Mer (FR)
(74) Mandataire: Siloret, Patrick
(86) Numéro de dépôt international: PCT/EP2014/053281
(87) Numéro de publication internationale: WO 2014/131680

(56) Documents cités:
- EP-A1- 1 918 208
- FR-A1- 2 951 949
- GB-A- 495 499
- US-A- 2 087 751

## Description

La présente invention concerne un procédé de décontamination par irradiation de l'intérieur d'une préforme.

L'invention concerne plus particulièrement un procédé de décontamination par irradiation de l'intérieur d'une préforme en matière thermoplastique destinée à la fabrication d'un récipient.

Dans le domaine des emballages agro-alimentaires, il est connu de réaliser un traitement de décontamination pour aseptiser ou stériliser au moins l'intérieur d'un récipient destiné à recevoir des denrées alimentaires.

Les traitements de décontamination appliqués ont pour but de détruire, ou à tout le moins de réduire, la présence d'organismes microbiologiques ou micro-organismes, tels qu'en particulier les germes, les bactéries, les spores, les moisissures, etc. afin de permettre la conservation des denrées.

Par récipient, on entend un corps creux tel que par exemple une bouteille, un flacon, un pot, etc., qui sont autant de récipients obtenus par transformation d'une préforme en matière thermoplastique qui est le plus souvent préalablement fabriquée par injection dans un moule.

Des matières thermoplastiques, le PET (Polyethylene Terephtalate) est le plus communément utilisé pour ces applications.

De manière connue, la préforme est successivement conditionnée thermiquement dans un four afin d'en ramollir la matière constitutive puis transformée en un récipient par soufflage dans un moule au moyen d'au moins un fluide sous pression, avec ou sans étirage.

En variante, la préforme injectée est directement transformée en récipient sans requérir alors de conditionnement thermique préalable.

Dans l'état de la technique, la décontamination d'une préforme est notamment réalisée par "voie chimique" au moyen d'un produit stérilisant tel que du peroxyde d'hydrogène (H₂O₂) ou autre produit présentant des propriétés bactéricide, virucide, fongicide, etc. analogues.

Le document WO-2006/136499 décrit par exemple un procédé de stérilisation de l'intérieur d'une préforme en matière thermoplastique au moyen du dépôt par condensation d'un film de buée de peroxyde d'hydrogène sur la surface interne de la préforme.

Bien que de tel procédé de décontamination donne satisfaction, on recherche cependant des solutions alternatives qui, sans produit stérilisant notamment, soient en particulier plus respectueuses de l'environnement, mais tout en conservant des niveaux de décontamination équivalents.

Une solution alternative possible consiste à réaliser la décontamination par irradiation par l'intermédiaire d'une source émettant un rayonnement ultraviolet, dit UV.

En effet, une telle décontamination par irradiation présente l'avantage de ne nécessiter l'emploi d'aucun produit chimique appliqué sur la préforme à décontaminer et ceci au bénéfice notamment d'une absence ultérieure d'éventuelles traces résiduelles dans le récipient final obtenu à partir de la préforme.

La décontamination par irradiation au moyen d'un rayonnement ultraviolet n'a cependant été jusqu'alors mise en oeuvre que pour traiter l'extérieur de la préforme.

A titre d'exemples non limitatifs de l'état de la technique, on se reportera au document WO-03/084818 qui décrit l'utilisation de rayonnement ultraviolet pour décontaminer les cols de préformes en matière thermoplastique ou encore au document WO-2008/049876 qui décrit la décontamination, dans un four, de la surface externe du corps de préformes par irradiation au moyen notamment d'un rayonnement ultraviolet.

L'une des raisons constituant un obstacle à l'utilisation de rayonnement ultraviolet pour décontaminer l'intérieur d'une préforme tient au fait que les matières thermoplastiques comme le PET ne laissent pas passer un tel un rayonnement ultraviolet.

Le rayonnement ultraviolet doit par conséquent irradier directement la surface à décontaminer, l'intérieur de la préforme ne pouvant être décontaminé par irradiation à travers la paroi de la préforme, depuis l'extérieur.

Le document FR-2.951.949 décrit un dispositif de décontamination comportant une source émettant un rayonnement ultraviolet qui est associée à un réflecteur destiné à réfléchir ledit rayonnement ultraviolet vers une zone de décontamination dans laquelle se trouve au moins un bouchon à décontaminer.

Le dispositif de décontamination selon ce document est destiné à la décontamination de bouchons, « classique » ou de type « sport ». Cependant, un tel dispositif n'est pas susceptible de permettre la décontamination de l'intérieur d'une préforme.

En effet, la décontamination de l'intérieur d'une préforme ne peut être obtenue avec un tel dispositif car le rayonnement réfléchi ne peut pas irradier l'ensemble de la surface interne d'une préforme. La décontamination de l'intérieur d'une préforme pose des problèmes différents de ceux pouvant exister avec un bouchon.

Dans une préforme, le corps est fermé à une extrémité par un fond de sorte que la seule ouverture communiquant avec l'intérieur de la préforme est constituée par celle que délimite le col de la préforme.

Par comparaison avec un bouchon, une préforme présente un corps creux de bien plus grande dimension or la décontamination doit être opérée sur toute la surface interne sans exception, depuis l'ouverture du col jusqu'au fond de la préforme et sur toute la hauteur du corps._Le but de la présente invention est notamment de proposer un nouveau procédé de décontamination par irradiation de l'intérieur d'une préforme qui soit simple, efficace et rapide et offre avantageusement une alternative à la décontamination par voie chimique.

Dans ce but l'invention propose un procédé de décontamination par irradiation de l'intérieur d'une préforme en matière thermoplastique destinée à la fabrication d'un récipient, ledit procédé étant défini dans la revendication 1.

Avantageusement, le procédé de décontamination est simple et rapide dès lors que l'ensemble des moyens de décontamination mis en oeuvre pour réaliser lesdites étapes demeurent en permanence à l'extérieur de la préforme, seul ledit au moins un rayonnement ultraviolet étant introduit à l'intérieur de la préforme grâce aux moyens de focalisation.

Par comparaison avec l'état de la technique, l'étape (c) au cours de laquelle ledit au moins un rayonnement ultraviolet est focalisé permet d'obtenir un faisceau qui pénètre par l'ouverture du col à l'intérieur de la préforme.

Avantageusement, les moyens de focalisation focalisent ledit au moins rayonnement ultraviolet émis puis réfléchi de manière à obtenir un faisceau concentrant ledit au moins rayonnement ultraviolet, lequel faisceau pénètre à l'intérieur de la préforme pour en irradier tout ou partie de la surface interne selon le mode de réalisation mis en oeuvre.

Avantageusement, les moyens de décontamination constitués par ladite au moins une source de rayonnement ultraviolet, les moyens réflecteurs et les moyens de focalisation ne sont pas introduits à l'intérieur de la préforme pour en décontaminer par irradiation de la surface interne.

Grâce à un tel agencement à l'extérieur des moyens de décontamination, le temps de traitement de l'intérieur d'une préforme correspond, en l'absence d'introduction et de retrait d'au moins une partie de ces moyens à l'intérieur de la préforme, quasi-totalement à une durée d'irradiation.

Avantageusement, le procédé de décontamination est particulièrement rapide et susceptible d'être mis en oeuvre dans une installation de fabrication de récipients stériles sans que cela n'impacte les cadences de fabrication.

De préférence, les moyens de décontamination sont alignés entre eux suivant un axe principal de la préforme et sont agencés à l'aplomb de la préforme, au dessus du col et de l'ouverture à travers laquelle est introduite ledit au moins un rayonnement ultraviolet.

Avantageusement, le procédé selon l'invention élimine tous risques de contamination de l'intérieur de la préforme par quelques moyens que ce soit.

En effet, de tels risques sont d'autant plus critiques que la décontamination est réalisée successivement sur plusieurs préformes appartenant à un flux comme par exemple le flux de préformes circulant dans une installation de fabrication de récipients stériles.

Avantageusement, la décontamination par irradiation est réalisée avant la transformation de la préforme en récipient.

En effet, la surface interne à décontaminer d'une préforme est inférieure à celle du récipient final (bouteille, flacon, pot, etc.) de sorte que la décontamination d'une préforme est plus économique, notamment grâce à une moindre consommation d'énergie.

De plus et par comparaison avec celle d'un récipient (comme une bouteille) qui est souvent ouvragée pour des raisons techniques et/ou esthétiques, la surface interne de la paroi d'une préforme ne comporte pas d'éléments en relief de sorte qu'aucun problème d'ombres et de surfaces masquées au rayonnement ne se pose avec une préforme.

Selon d'autres caractéristiques de l'invention :
- l'étape (b) consiste à réfléchir ledit au moins un rayonnement ultraviolet émis par l'intermédiaire de moyens réflecteurs qui sont agencés, par rapport à ladite au moins une source, pour diriger ledit au moins un rayonnement ultraviolet émis en direction du col d'une préforme ;
- l'étape (c) consiste à concentrer ledit au moins un rayonnement ultraviolet réfléchi en le focalisant à l'intérieur de la préforme avec des moyens de focalisation qui sont interposés entre lesdits moyens réflecteurs et le col de la préforme ;
- le procédé comporte une première séquence de décontamination de l'intérieur de la préforme consistant à réaliser successivement lesdites étapes (a), (b) et (c) et au moins une deuxième séquence de décontamination consistant à réaliser à nouveau lesdites étapes (a), (b) et (c) ;
- le procédé comporte, entre lesdites première et deuxième séquences de décontamination, au moins une étape intermédiaire de temporisation d'une durée déterminée au cours de laquelle l'irradiation de l'intérieur de la préforme est interrompue ;
- la première séquence de décontamination consiste à irradier l'intérieur de la préforme avec un rayonnement ultraviolet qui présente au moins une raie principale ayant une longueur d'onde donnée et la deuxième séquence de décontamination consiste à irradier l'intérieur de ladite préforme avec un rayonnement ultraviolet qui présente au moins une raie principale ayant une longueur d'onde donnée identique à celle de la première séquence ou différente de celle de la première séquence ;
- la première séquence de décontamination consiste à irradier une première zone définie à l'intérieur de la préforme et la deuxième séquence de décontamination consiste à irradier une deuxième zone définie à l'intérieur de ladite préforme, ladite deuxième zone étant distincte de la première zone ;
- la première séquence de décontamination consiste à irradier la première zone définie à l'intérieur de la préforme avec un rayonnement ultraviolet qui présente au moins une raie principale ayant une longueur d'onde donnée et la deuxième séquence de décontamination consiste à irradier la deuxième zone définie à l'intérieur de ladite préforme avec un rayonnement ultraviolet qui présente au moins une raie principale ayant une longueur d'onde donnée identique à celle de la première séquence ; ;
- l'un des rayonnements ultraviolets est de type "C" et présente au moins une raie principale ayant une longueur d'onde comprise entre 100 nm et 280 nm ;
- l'un des rayonnements ultraviolets est de type "A" et présente au moins une raie principale ayant une longueur d'onde comprise entre 315 nm et 400 nm.

D'autres caractéristiques et avantages de l'invention apparaîtront à la lecture de la description détaillée qui suit pour la compréhension de laquelle on se reportera aux dessins annexés dans lesquels :
- la figure 1 est une vue en coupe qui représente schématiquement un exemple de dispositif de décontamination pour la mise en oeuvre du procédé de décontamination de l'intérieur d'une préforme selon l'invention ;
- la figure 2 est une vue de dessus qui représente schématiquement un exemple d'agencement des moyens de décontamination dans une machine de décontamination.

L'invention concerne un procédé de décontamination de l'intérieur d'une préforme en matière thermoplastique, telle que le PET, et qui est destinée à la fabrication d'un récipient, notamment mais non exclusivement une bouteille.

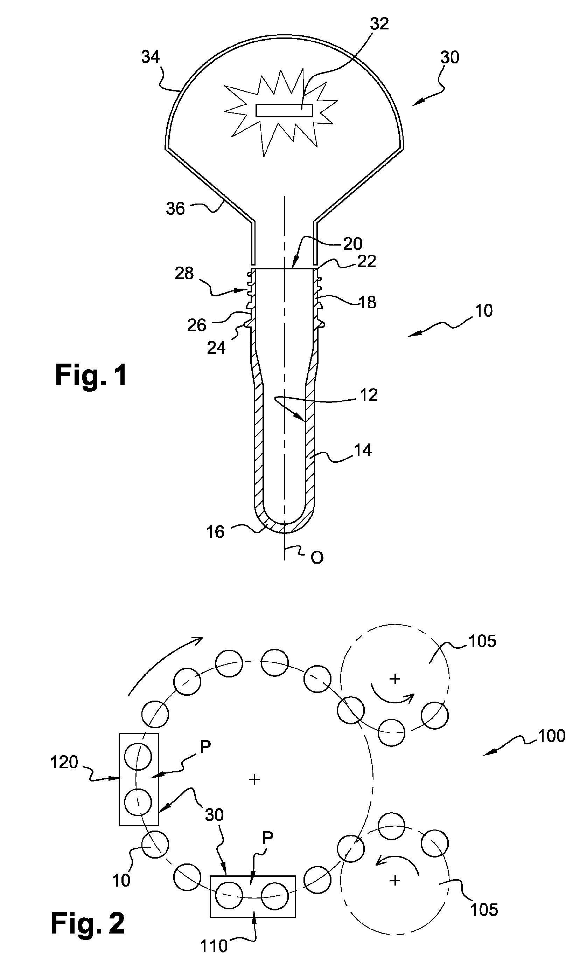

On a représenté à la figure 1, à titre non limitatif, un exemple d'une préforme 10 en matière thermoplastique.

Une telle préforme est généralement obtenue par injection de matière plastique et présente des caractéristiques (dimensions, répartition de la matière, etc.) qui sont déterminées par le récipient final à obtenir, en particulier sa forme ou encore sa contenance.

La préforme 10 comporte une surface 12 interne délimitée par un corps 14 fermé à une extrémité par un fond 16 et qui, à l'autre extrémité, comporte un col 18 délimitant une ouverture 20 d'accès à l'intérieur de la préforme 10.

Le col 18 de la préforme 10 présente sa forme définitive à l'issue de la fabrication par injection de la préforme et correspond au col du récipient final, un bord 22 du col (ou buvant) délimite circonférentiellement l'ouverture 20 circulaire constituant le seul accès à l'intérieur de la préforme 10.

Dans l'exemple représenté à la figure 1, le col 18 comporte une collerette 24 qui s'étend radialement en saillie vers l'extérieur et une gorge 26 annulaire qui, adjacente à ladite collerette 24 est destinée à recevoir ultérieurement une bague d'inviolabilité.

Une telle bague est destinée à garantir au consommateur l'intégrité du récipient final rempli avant une première ouverture.

De préférence, le col 18 comporte un filetage 28 destiné à permettre la fermeture du récipient final par un bouchon à vis complémentaire.

Conformément à l'invention, le procédé de décontamination par irradiation de l'intérieur d'une préforme 10 en matière thermoplastique destinée à la fabrication d'un récipient comporte au moins les étapes consistant successivement à :
(a) - commander l'émission d'au moins un rayonnement ultraviolet par au moins une source ;
(b) - réfléchir ledit au moins un rayonnement ultraviolet émis pour le diriger en direction du col 18 d'une préforme 10 ;
(c) - concentrer ledit au moins un rayonnement ultraviolet réfléchi en le focalisant pour obtenir un faisceau qui pénètre à l'intérieur de la préforme 10.

Avantageusement, le procédé est susceptible d'être mis en oeuvre au moyen d'un dispositif 30 de décontamination comportant des moyens de décontamination qui sont agencés à l'extérieur de ladite préforme et qui sont aptes à irradier au moyen d'un rayonnement ultraviolet au moins l'intérieur de la préforme.

On a représenté à la figure 1 de manière schématique un exemple de réalisation d'un tel dispositif 30 de décontamination.

Avantageusement, lesdits moyens du dispositif 30 de décontamination comportent au moins une source 32 qui est apte à émettre au moins un rayonnement ultraviolet pour réaliser l'étape (a) selon le procédé.

De préférence, la source 32 est une lampe à rayonnement ultraviolet (UV) comme par exemple une lampe au xénon ou encore une lampe à vapeur de mercure, ledit rayonnement ultraviolet (UV) pouvant être émis de manière continue ou discontinue.

De préférence, le rayonnement ultraviolet de la source 32 est au moins de type "C", c'est-à-dire un rayonnement "UVC" qui présente au moins une raie principale ayant une longueur d'onde comprise entre 100 nm et 280 nm.

Avantageusement, la longueur d'onde du rayonnement "UVC" est alors comprise entre 250 nm et 275 nm, en particulier égale à 265 nm.

En variante, le rayonnement ultraviolet de la source 32 est de type "A", c'est-à-dire un rayonnement "UVA" qui présente au moins une raie principale ayant une longueur d'onde comprise entre 315 nm et 400 nm.

Avantageusement, la source 32 est multiple de manière à émettre une combinaison rayonnante de rayonnements ultraviolets comportant des "UVC" et des "UVA".

Avantageusement, lesdits moyens du dispositif 30 de décontamination comportent au moins des moyens 34 réflecteurs qui, associés à ladite au moins une source 32, sont aptes à réfléchir ledit au moins un rayonnement ultraviolet émis pour le diriger en direction de la préforme 10, notamment du col 18 de la préforme 10.

Les moyens 34 réflecteurs comportent par exemple au moins un miroir présentant une surface réfléchissante concave apte diriger ledit au moins un rayonnement ultraviolet (UV) en direction du col 18 de la préforme 10.

De préférence, lesdits moyens du dispositif 30 de décontamination comportent encore au moins des moyens 36 de focalisation qui sont interposés entre lesdits moyens 34 réflecteurs et le col 18 de la préforme 10.

Avantageusement, lesdits moyens 36 de focalisation sont aptes à concentrer ledit au moins un rayonnement ultraviolet (UV) en le focalisant, à travers l'ouverture 20, à l'intérieur de la préforme 10 pour en décontaminer la surface 12 interne par irradiation.

Les moyens 36 de focalisation comportent par exemple au moins un collecteur ou encore au moins deux condenseurs asphériques complémentaires, lesdits moyens 36 de focalisation délivrant en sortie un faisceau qui pénètre à l'intérieur de la préforme 10.

Le faisceau obtenu grâce aux moyens 36 de focalisation est par exemple un faisceau convergent.

De préférence, le dispositif 30 de décontamination comporte des moyens de refroidissement (non représentés) qui sont aptes à refroidir tout ou partie des moyens de décontamination dudit dispositif, notamment les moyens 34 réflecteurs.

Dans un premier mode de réalisation mettant en oeuvre le procédé de décontamination selon l'invention, le procédé comporte au moins une séquence de décontamination consistant à réaliser successivement lesdites étapes (a), (b) et (c) pour décontaminer en une fois l'intérieur de la préforme 10.

Avantageusement, ladite séquence de décontamination comportant lesdites étapes (a), (b) et (c) est répétée au moins deux fois.

Le procédé comporte alors une première séquence de décontamination de l'intérieur de la préforme 10 consistant à réaliser successivement lesdites étapes (a), (b) et (c) et au moins une deuxième séquence de décontamination consistant à réaliser à nouveau lesdites étapes (a), (b) et (c).

De préférence, le procédé comporte, entre lesdites première et deuxième séquences de décontamination, au moins une étape intermédiaire de temporisation d'une durée déterminée au cours de laquelle l'irradiation de l'intérieur de la préforme 10 est interrompue.

Selon un premier exemple, la première séquence de décontamination consiste à irradier l'intérieur de la préforme 10 avec un rayonnement ultraviolet qui présente au moins une raie principale ayant une longueur d'onde donnée, telle que des UVC de longueur d'onde comprise entre 100 nm et 280 nm.

La deuxième séquence de décontamination consiste à irradier l'intérieur de ladite préforme 10 avec un rayonnement ultraviolet qui présente au moins une raie principale ayant une longueur d'onde donnée identique à celle de la première séquence.

Selon un autre exemple, la séquence de décontamination est répétée pour irradier la surface 12 interne de la préforme 10 avec successivement des rayonnements ultraviolets de type différent, par exemple des UVC puis des UVA.

La première séquence de décontamination consiste alors à irradier l'intérieur de la préforme avec un rayonnement ultraviolet qui présente au moins une raie principale ayant une longueur d'onde donnée, de préférence des UVC de longueur d'onde comprise entre 100 nm et 280 nm.

La deuxième séquence de décontamination consiste à irradier l'intérieur de ladite préforme 10 avec un rayonnement ultraviolet qui présente au moins une raie principale ayant une longueur d'onde donnée différente de celle de la première séquence, de préférence des UVA de longueur d'onde comprise entre 315 nm et 400 nm. L'utilisation de rayonnements ultraviolets de type différent permet d'obtenir une meilleure décontamination en permettant la destruction d'une plus grande variété de microorganismes.

Selon un deuxième mode de réalisation mettant en oeuvre le procédé de décontamination selon l'invention, le procédé comporte :
- une première séquence de décontamination de l'intérieur de la préforme consistant à réaliser successivement lesdites étapes (a), (b) et (c) et au moins
- une deuxième séquence de décontamination consistant à réaliser à nouveau lesdites étapes (a), (b) et (c).

De préférence, le procédé comporte, entre lesdites première et deuxième séquences de décontamination, au moins une étape intermédiaire de temporisation d'une durée déterminée au cours de laquelle l'irradiation de l'intérieur de la préforme est interrompue.

Tel qu'indiqué précédemment pour le premier mode de réalisation, les première et deuxième séquences peuvent permettre d'irradier deux fois l'intérieur de la préforme 10, soit toute la surface 12 interne de la préforme 10.

Selon l'invention, les première et deuxième séquences sont de préférence réalisées pour irradier successivement deux portions complémentaires qui, réunies ensemble, correspondent au moins à ladite surface 12 interne de la préforme 10.

Avantageusement, la surface 12 interne de la préforme 10 est par conséquent irradiée non pas en une seule fois (même plusieurs fois de suite) mais au moins en deux fois.

Selon l'invention, la première séquence de décontamination consiste à irradier une première zone définie à l'intérieur de la préforme tandis que la deuxième séquence de décontamination consiste à irradier une deuxième zone définie à l'intérieur de ladite préforme, ladite deuxième zone étant distincte de la première zone.

A titre d'exemple non limitatif, ladite première zone définie à l'intérieur de la préforme comporte au moins la portion de surface 12 interne entourée par le col 18 et se prolonge au moins jusqu'au tronçon du corps 14 adjacent à la collerette 24 dudit col 18 et présentant ici en coupe une forme tronconique.

La deuxième zone définie à l'intérieur de la préforme est alors avantageusement formée par la portion complémentaire et restante de la surface 12 interne qui s'étend axialement suivant l'axe O jusqu'au fond 16.

Lesdites première zone et deuxième zone définies à l'intérieur de la préforme 10 sont complémentaires.

Avantageusement, la séparation de la surface 12 interne de la préforme 10 en au moins une première et une deuxième zones permet d'optimiser les moyens de décontamination en vue de l'irradiation de chacune desdites zones.

Tel qu'indiqué précédemment, chacune desdites première et deuxième zones à l'intérieur de la préforme 10 est susceptible d'être irradiée par une source 32 émettant au moins un rayonnement UV d'un type déterminé, en particulier au moins des UVC.

Selon cet exemple, la première séquence de décontamination consiste à irradier la première zone définie à l'intérieur de la préforme 10 avec un rayonnement ultraviolet qui présente au moins une raie principale ayant une longueur d'onde donnée, telle que au moins des UVC, et la deuxième séquence de décontamination consiste à irradier la deuxième zone définie à l'intérieur de ladite préforme 10 avec un rayonnement ultraviolet qui présente au moins une raie principale ayant une longueur d'onde donnée identique à celle de la première séquence, telle que au moins des UVC.

De préférence, lesdites première et deuxième zones à l'intérieur de la préforme 10 sont irradiées simultanément par une source 32 émettant une combinaison irradiante comportant par exemple des UVC et des UVA.

Grâce à une telle combinaison irradiante de différents types de rayonnements ultraviolets telle que des UVC et des UVA, on obtient une meilleure décontamination en raison de la destruction d'une plus grande variété de microorganismes.

En variante, lesdites première et deuxième zones à l'intérieur de la préforme 10 pourraient chacune être irradiées successivement par une première source 32 émettant des UVC puis par une deuxième source émettant des UVA.

Avantageusement, la première séquence de décontamination consiste à irradier au moins une partie de l'intérieur de la préforme 10, telle que la première zone précitée de la surface 12 interne comportant notamment celle du col 18 et cela par l'application, selon la source 32, d'au moins un rayonnement ultraviolet (UV) présentant au moins une raie principale ayant une longueur d'onde donnée.

Avantageusement, la deuxième séquence de décontamination consiste à irradier au moins une partie de l'intérieur de la préforme 10 telle que la deuxième zone précitée de la surface 12 interne comportant notamment celle du fond 16, et cela par l'application, selon la source 32, d'au moins un rayonnement ultraviolet (UV) présentant au moins une raie principale ayant de préférence la même longueur d'onde.

On a représenté à la figure 2, une machine 100 de décontamination destinée à mettre en oeuvre le procédé de décontamination selon l'invention.

La machine 100 de décontamination est notamment mais non exclusivement susceptible de permettre la mise en oeuvre du procédé de décontamination selon les exemples de modes de réalisation décrits précédemment.

La machine 100 comporte au moins un poste P comportant les moyens du dispositif 30 de décontamination nécessaires à la mise en oeuvre des étapes (a), (b) et (c) selon le procédé de décontamination de l'invention.

Avantageusement, un poste P comporte un dispositif 30 de décontamination et par conséquent au moins une source 32 associée à des moyens 34 réflecteurs et des moyens 36 de focalisation.

De préférence, la machine 100 comporte plusieurs postes P de décontamination et tel que représenté à la figure 2 les postes P sont répartis sur une partie de la circonférence de la machine qui est ici de type rotative.

En variante, les postes P peuvent être agencés en ligne pour constituer une machine de type linéaire.

Des moyens 105 de transfert sont avantageusement associés à ladite machine 100 pour réaliser respectivement l'alimentation en préformes 10 à décontaminer et l'évacuation des préformes 10 décontaminées.

Les moyens 105 de transfert sont par exemple réalisés sous la forme d'une roue à encoches dans chacune desquelles une préforme 10 est susceptible d'être reçue et maintenue par l'intermédiaire de sa collerette 24 ou encore sont réalisés sous la forme de pinces notamment aptes à saisir la préforme 10 au niveau de la gorge annulaire 26.

De préférence, les postes P sont fixes et seules les préformes 10 sont déplacées suivant un parcours de décontamination circulaire.

Avantageusement, la machine 100 comporte au moins une première zone 110 comportant au moins un poste P et au moins une deuxième zone 120 comportant au moins un poste P.

De préférence chaque zone 110, 120 comporte au moins deux postes pour la décontamination simultanée de plus d'une préforme 10.

Dans le cas du premier mode de réalisation, les postes P de chacune desdites première et deuxième zones 110 et 120 permettent de réaliser deux fois les étapes (a), (b) et (c) pour irradier la surface 12 interne d'une préforme 10.

Selon les exemples décrits précédemment, les postes P de chacune desdites première et deuxième zones 110 et 120 comportent une source identique émettant un rayonnement ultraviolet de même longueur d'onde, de préférence des UVC.

En variante, les postes P de chacune desdites première et deuxième zones 110 et 120 comportent une source différente, l'une émettant un rayonnement ultraviolet d'une longueur d'onde donnée, de préférence des UVC, tandis que l'autre émet un rayonnement ultraviolet d'une autre longueur d'onde donnée, de préférence des UVA.

La machine 100 peut également comporter plusieurs zones de traitement pour accroître la capacité de décontamination de préformes par unité de temps.

Avantageusement et selon le deuxième mode de réalisation décrit précédemment, les postes P de chacune desdites première et deuxième zones 110 et 120 comportent respectivement des moyens de décontamination qui sont différents.

La première zone 110 correspond par exemple à la première séquence de décontamination au cours de laquelle les étapes (a), (b) et (c) sont mises en oeuvre au moins une fois pour décontaminer une première zone définie de la préforme 10.

La deuxième zone 120 correspond alors à la deuxième séquence de décontamination au cours de laquelle les étapes (a), (b) et (c) sont mises en oeuvre au moins une fois pour décontaminer une deuxième zone définie de la préforme 10, distincte de la première zone.

Les première et deuxième zones 110, 120 ne sont ici pas consécutives pour effectuer ladite étape intermédiaire de temporisation.

La première zone définie de la préforme 10 est décontaminée dans la première zone 110 de la machine 100, par exemple avec au moins des UVC ou en variante avec une combinaison d'UVC et d'UVA.

De la même manière, la deuxième zone définie de la préforme 10 est décontaminée dans la deuxième zone 120 de la machine 100, par exemple avec au moins des UVC ou en variante avec une combinaison d'UVC et d'UVA.

Bien entendu, la machine 100 qui vient d'être décrite ne constitue qu'un exemple non limitatif de machine comportant au moins un dispositif 30 de décontamination pour la mise en oeuvre unique ou répétée des étapes (a), (b) et (c) du procédé de décontamination selon l'invention.

La présente invention est susceptible d'application industrielle et propose un procédé de décontamination par irradiation de l'intérieur d'une préforme 10 en matière thermoplastique destinée à la fabrication d'un récipient.

## Revendications

1. Procédé de décontamination par irradiation de l'intérieur d'une préforme (10) en matière thermoplastique destinée à la fabrication d'un récipient, ledit procédé comportant au moins les étapes consistant successivement à :
(a) - commander l'émission d'au moins un rayonnement ultraviolet (UV) par au moins une source (32) ;
(b) - réfléchir ledit au moins un rayonnement ultraviolet (UV) émis pour le diriger en direction du col (18) d'une préforme (10) ;
(c) - concentrer ledit au moins un rayonnement ultraviolet (UV) réfléchi en le focalisant pour obtenir un faisceau qui pénètre à l'intérieur de la préforme (10),
ledit procédé comportant :
- une première séquence de décontamination de l'intérieur de la préforme (10) consistant à réaliser successivement lesdites étapes (a), (b) et (c) et à irradier une première zone définie à l'intérieur de la préforme (10) et au moins
- une deuxième séquence de décontamination consistant à réaliser à nouveau lesdites étapes (a), (b) et (c) et à irradier une deuxième zone définie à l'intérieur de ladite préforme (10), ladite deuxième zone étant distincte de la première zone.

2. Procédé selon la revendication 1, **caractérisé en ce que** l'étape (b) consiste à réfléchir ledit au moins un rayonnement ultraviolet (UV) émis par l'intermédiaire de moyens (34) réflecteurs qui sont agencés, par rapport à ladite au moins une source (32), pour diriger ledit au moins un rayonnement ultraviolet (UV) émis en direction du col (18) d'une préforme (10).

3. Procédé selon la revendication 2, **caractérisé en ce que** l'étape (c) consiste à concentrer ledit au moins un rayonnement ultraviolet (UV) réfléchi en le focalisant à l'intérieur de la préforme (10) avec des moyens (36) de focalisation qui sont interposés entre lesdits moyens (34) réflecteurs et le col (18) de la préforme (10).

4. Procédé selon l'une quelconque des revendications 1 à 3, **caractérisé en ce que** lesdites première et deuxième zones à l'intérieur de la préforme (10) sont irradiées simultanément par une source (32) émettant une combinaison irradiante.

5. Procédé selon l'une quelconque des revendications 1 à 3, **caractérisé en ce que** lesdites première et deuxième zones à l'intérieur de la préforme (10) sont chacune irradiées successivement par une première source (32) émettant des UVC puis par une deuxième source émettant des UVA.

6. Procédé selon la revendication 1, **caractérisé en ce que** le procédé comporte, entre lesdites première et deuxième séquences de décontamination, au moins une étape intermédiaire de temporisation d'une durée déterminée au cours de laquelle l'irradiation de l'intérieur de la préforme (10) est interrompue.

7. Procédé selon l'une des revendications 1 à 3, **caractérisé en ce que** la première séquence de décontamination consiste à irradier la première zone définie à l'intérieur de la préforme (10) avec un rayonnement ultraviolet qui présente au moins une raie principale ayant une longueur d'onde donnée et **en ce que** la deuxième séquence de décontamination consiste à irradier la deuxième zone définie à l'intérieur de ladite préforme (10) avec un rayonnement ultraviolet qui présente au moins une raie principale ayant une longueur d'onde donnée identique à celle de la première séquence.

8. Procédé selon la revendication 7, **caractérisé en ce que** l'un des rayonnements ultraviolets est de type "C" et présente au moins une raie principale ayant une longueur d'onde comprise entre 100 nm et 280 nm.

9. Procédé selon la revendication 7, **caractérisé en ce que** l'un des rayonnements ultraviolets est de type "A" et présente au moins une raie principale ayant une longueur d'onde comprise entre 315 nm et 400 nm.

## Patentansprüche

1. Verfahren zur Dekontamination des Inneren eines Vorformlings (10) aus thermoplastischem Material durch Bestrahlung, der für die Herstellung eines Behälters bestimmt ist, wobei das Verfahren mindestens die folgenden Schritte umfasst, die aufeinanderfolgend bestehen aus:
(a) Steuern der Emission mindestens einer Ultraviolett(UV)-Strahlung durch mindestens eine Quelle (32);
(b) Reflektieren der mindestens einen emittierten Ultraviolett(UV)-Strahlung, um diese in die Richtung des Halses (18) eines Vorformlings (10) zu richten;
(c) Konzentrieren der mindestens einen reflektierten Ultraviolett(UV)-Strahlung, indem diese fokussiert wird, um ein Bündel zu erhalten, das in das Innere des Vorformlings (10) eindringt,
wobei das Verfahren umfasst:
- eine erste Sequenz der Dekontamination des Inneren des Vorformlings (10), die aus der aufeinanderfolgenden Durchführung der Schritte (a), (b) und (c) und aus der Bestrahlung einer ersten Zone, die im Inneren des Vorformlings (10) definiert ist, besteht, und mindestens
- eine zweite Sequenz der Dekontamination, die aus der erneuten Durchführung der Schritte (a), (b) und (c) und aus der Bestrahlung einer zweiten Zone, die im Inneren des Vorformlings (10) definiert ist, besteht, wobei die zweite Zone von der ersten Zone verschieden ist.

2. Verfahren nach Anspruch 1,
**dadurch gekennzeichnet, dass** der Schritt (b) aus dem Reflektieren der mindestens einen Ultraviolett(UV)-Strahlung besteht, welche von Reflektormitteln (34) emittiert wird, die in Bezug auf die mindestens eine Quelle (32) ausgelegt sind, die mindestens eine emittierte Ultraviolett(UV)-Strahlung in die Richtung des Halses (18) eines Vorformlings (10) zu richten.

3. Verfahren nach Anspruch 2,
**dadurch gekennzeichnet, dass** der Schritt (c) aus dem Konzentrieren der mindestens einen reflektierten Ultraviolett(UV)-Strahlung besteht, indem diese in das Innere des Vorformlings (10) mit Fokussierungsmitteln (36) fokussiert wird, die zwischen den Reflektormitteln (34) und dem Hals (18) des Vorformlings (10) angeordnet sind.

4. Verfahren nach einem der Ansprüche 1 bis 3,
**dadurch gekennzeichnet, dass** die erste und zweite Zone im Inneren des Vorformlings (10) gleichzeitig von einer Quelle (32) bestrahlt werden, die eine Strahlungskombination emittiert.

5. Verfahren nach einem der Ansprüche 1 bis 3,
**dadurch gekennzeichnet, dass** die erste und zweite Zone im Inneren des Vorformlings (10) jeweils aufeinanderfolgend von einer ersten Quelle (32), die UVC emittiert, und dann von einer zweiten Quelle, die UVA emittiert, bestrahlt werden.

6. Verfahren nach Anspruch 1,
**dadurch gekennzeichnet, dass** das Verfahren, zwischen der ersten und zweiten Sequenz der Dekontamination, mindestens einen Zwischenwarteschritt mit einer vorherbestimmten Dauer umfasst, während welcher die Bestrahlung des Inneren des Vorformlings (10) unterbrochen wird.

7. Verfahren nach einem der Ansprüche 1 bis 3,
**dadurch gekennzeichnet, dass** die erste Sequenz der Dekontamination aus dem Bestrahlen der ersten Zone, welche im Inneren des Vorformlings (10) definiert ist, mit einer Ultraviolett-Strahlung, die mindestens einen Hauptstrahl mit einer gegebenen Wellenlänge aufweist, besteht, und dass die zweite Sequenz der Dekontamination aus dem Bestrahlen der zweiten Zone, welche im Inneren des Vorformlings (10) definiert ist, mit einer Ultraviolett-Strahlung, die mindestens einen Hauptstrahl mit einer gegebenen Wellenlänge aufweist, die mit jener der ersten Sequenz identisch ist, besteht.

8. Verfahren nach Anspruch 7,
**dadurch gekennzeichnet, dass** eine der Ultraviolett-Strahlungen vom Typ "C" ist und mindestens einen Hauptstrahl mit einer Wellenlänge zwischen 100 nm und 280 nm aufweist.

9. Verfahren nach Anspruch 7,
**dadurch gekennzeichnet, dass** eine der Ultraviolett-Strahlungen vom Typ "A" ist und mindestens einen Hauptstrahl mit einer Wellenlänge zwischen 315 nm und 400 nm aufweist.

## Claims

1. Method for decontaminating, by irradiation from the interior, a preform (10) made of thermoplastic and intended for the manufacture of a container, said method including at least steps consisting in, in succession:
(a) commanding the emission of at least one ultraviolet (UV) emission from at least one source (32);
(b) reflecting said at least one emitted ultraviolet (UV) emission in order to direct it toward the neck (18) of a preform (10); and
(c) concentrating said at least one reflected ultraviolet (UV) emission by focusing it to obtain a beam that penetrates into the interior of the preform (10),
said method including:
- a first sequence for decontaminating the interior of the preform (10), consisting in carrying out said steps (a), (b) and (c) in succession and in irradiating a first defined zone in the interior of the preform (10); and
- at least one second decontaminating sequence consisting in carrying out said steps (a), (b) and (c) once again and in irradiating a second defined zone in the interior of said preform (10), said second zone being separate from the first zone.

2. Method according to Claim 1, **characterized in that** step (b) consists in reflecting said at least one emitted ultraviolet (UV) emission via reflective means (34) that are arranged, with respect to said at least one source (32), to direct said at least one emitted ultraviolet (UV) emission toward the neck (18) of a preform (10).

3. Method according to Claim 2, **characterized in that** step (c) consists in concentrating said at least one reflected ultraviolet (UV) emission by focusing it into the interior of the preform (10) with focusing means (36) that are interposed between said reflective means (34) and the neck (18) of the preform (10).

4. Method according to any one of Claims 1 to 3, **characterized in that** said first and second zones in the interior of the preform (10) are simultaneously irradiated by a source (32) emitting an irradiating combination.

5. Method according to any one of Claims 1 to 3, **characterized in that** said first and second zones in the interior of the preform (10) are each irradiated in succession by a first source (32) emitting in the UVC then by a second source emitting in the UVA.

6. Method according to Claim 1, **characterized in that** the method includes, between said first and second decontaminating sequences, at least one intermediate step of waiting a set time during which the irradiation of the interior of the preform (10) is interrupted.

7. Method according to one of Claims 1 to 3, **characterized in that** the first decontaminating sequence consists in irradiating the first defined zone in the interior of the preform (10) with an ultraviolet emission that contains at least one main spectral line having a given wavelength, and **in that** the second decontaminating sequence consists in irradiating the second defined zone in the interior of said preform (10) with an ultraviolet emission that contains at least one main spectral line having a given wavelength identical to that of the first sequence.

8. Method according to Claim 7, **characterized in that** one of the ultraviolet emissions is of type "C" and contains at least one main spectral line having a wavelength comprised between 100 nm and 280 nm.

9. Method according to Claim 7, **characterized in that** one of the ultraviolet emissions is of type "A" and contains at least one main spectral line having a wavelength comprised between 315 nm and 400 nm.
